(19) Europäisches Patentamt
European Patent Office
Office européen des brevets

(11) **EP 1 757 362 A1**

(12) **EUROPEAN PATENT APPLICATION**
published in accordance with Art. 158(3) EPC

(43) Date of publication:
**28.02.2007 Bulletin 2007/09**

(51) Int Cl.:
***B01J 20/20*** (2006.01)   ***A61K 31/775*** (2006.01)
***B01J 20/30*** (2006.01)

(21) Application number: **05743916.8**

(22) Date of filing: **27.05.2005**

(86) International application number:
**PCT/JP2005/009748**

(87) International publication number:
**WO 2005/115611 (08.12.2005 Gazette 2005/49)**

(84) Designated Contracting States:
**AT BE BG CH CY CZ DE DK EE ES FI FR GB GR HU IE IS IT LI LT LU MC NL PL PT RO SE SI SK TR**

(30) Priority: **31.05.2004 JP 2004160959**

(71) Applicant: **Teikoku Medix Co., Ltd.**
**Saitama-shi**
**Saitama 331-0056 (JP)**

(72) Inventors:
• **HAMASAKI, Isao,**
**JAPAN ENVIROCHEMICALS, LTD.**
**Osaka-shi,**
**Osaka 5320024 (JP)**
• **YANAGI, Juichi,**
**JAPAN ENVIROCHEMICALS, LTD.**
**Osaka-shi,**
**Osaka 5320024 (JP)**

• **MOURI, Motoya,**
**JAPAN ENVIROCHEMICALS, LTD.**
**Osaka-shi,**
**Osaka 5320024 (JP)**
• **SAITO, Mitsuhiro,**
**OHTA PHARMACEUTICAL CO., LTD.**
**Saitama-shi,**
**Saitama 3310056 (JP)**
• **MIZUNOYA, Takao,**
**OHTA PHARMACEUTICAL CO., LTD.**
**Saitama-shi,**
**Saitama 3310056 (JP)**
• **SHIMIZU, Toshio,**
**OHTA PHARMACEUTICAL CO., LTD.**
**Saitama-shi,**
**Saitama 3310056 (JP)**

(74) Representative: **von Kreisler, Alek**
**Patentanwälte**
**von Kreisler-Selting-Werner**
**Postfach 10 22 41**
**D-50462 Köln (DE)**

(54) **ADSORBENT AND PROCESS FOR PRODUCING THE SAME**

(57)     An object of the invention is to provide an adsorbent having high adsorbability per unit volume and a high adsorption rate as an adsorbent for water treatment or medical care and to provide an adsorbent capable of completely adsorbing an aimed substance within a short time as an adsorbent for medical care.

The desired adsorbent is an adsorbent produced from a phenol resin as a starting material and having 0.10 to 0.60 ml/g of volume of fine pores with diameters in a range from 0.02 to 10 µm, a specific surface area in a range from 800 to 3000 $m^2$/g, and an average particle diameter in a range from 0.1 to 0.8 mm. The adsorbent is produced by conducting carbonization of the phenol resin as a starting material at 350 to 550°C, then heating up to 700 to 950°C at a rate of 5 to 20°C/min, and successively carrying out activation of the obtained carbonized product.

EP 1 757 362 A1

**Description**

Technical Field

The invention relates to an adsorbent having a very high adsorption rate and adsorption capacity per unit volume and its production method. Particularly, the invention relates to an adsorbent specially suitable for water treatment and medical use and capable of efficiently removing an object to be adsorbed such as impurities in a short contact time and its production method.

Background Art

In general, since being packed in such an apparatus or a facility having a constant volume as a column, an adsorption container and an adsorption pond, an adsorbent is required to have not only high adsorbability per unit weight but also high adsorbability per unit volume. Further, generally the time for contact of a liquid such as water containing an object substance to be adsorbed with an adsorbent in adsorption operation is limited. If the adsorption rate is slow, the adsorbent cannot be used practically for an aimed purpose.
To solve the above-mentioned matter, it is supposed to be possible to make the particle diameter of the adsorbent small, however if the particle diameter is made small, the water flow resistance is increased and the bulk density is lowered and thus a method for supporting the adsorbent and the way of handling the adsorbent have to be specially contrived to deal with the matter well and it is keenly required to develop an adsorbent having high adsorbability per unit volume and quick adsorption rate.
Particularly, in the case of using an adsorbent for medical care, unless a toxic object substance to be adsorbed is quickly adsorbed and removed in digestive tracts, the toxic substance is to be adsorbed internally in bodies, and accordingly no efficacy can be expected unless the adsorbent has a high adsorption rate.
For example, an adsorbent obtained by activating petroleum pitch and having 0.1 to 1.0 ml/g of volume of pores with the pore radius in a range from 100 to 75000 Å and its production method are proposed (Patent Document No. 1), however those produced actually have many pores with so small diameters that the pore volume of pores with the diameter of 80 Å (8 nm) or smaller is in a range from 0.70 to 0.75 ml/g and the pore volume of pores with the diameter of 37.5 to 75000 Å (3.75 to 7500 nm) is in a range from 0.20 to 0.23 ml/g and the meaning of the critical numeral restriction for the fine pore volume is not made clear and there is no description on the adsorption rate.
Further, an adsorbent produced from petroleum pitch as a starting material and having not less than 0.04 ml/g and less than 0.10 ml/g of the volume of pores with the pore diameter in a range from 20 to 15000 nm is proposed (Patent Document No. 2), however this adsorbent also has many fine pores with very small diameters and the description only refers to the selectivity of an object substance to be adsorbed in the equilibrium adsorption state and no description regarding to the adsorption rate is given.
There is another document (Patent Document No. 3) describing an adsorbent produced from a phenol resin as a starting material and having 0.04 ml/g or 0.06 ml/g of the pore volume of pores with diameters in a range from 7.5 to 15000 nm and the adsorbent is also an adsorbent having many fine pores with small diameters, however the description only refers to the selectivity of an object substance to be adsorbed in the equilibrium adsorption state and any description regarding to the adsorption rate is not at all given.
Further, an activated carbon having 0.04 ml/g or lower total pore volume ratio of fine pores with pore diameters in a range from 20 to 10000 nm is described (Patent Document No. 4), however there is also no description regarding the adsorption ratio of activated carbon.

Patent Document No. 1: Japanese Patent Application Laid-Open (JP-A) No. S 62-11611
Patent Document No. 2: JP-A No. 2002-308785
Patent Document No. 3: WO No. 2004-039381A1
Patent Document No. 4: JP-A No. 2004-244414

Disclosure of the Invention

Problem to be solved by the Invention

The purpose of the invention is to provide an adsorbent capable of adsorbing and removing a large quantity of an impurity such as a coloring material, an organic or inorganic toxic substance contaminating water, a toxic substance or a low molecular weight metabolism product existing in digestive tracts of human being and animals within a short time.

Means for Solving the Problem

A phenol resin is a polymer substance obtained by condensation of a phenol and an aldehyde and is condensed. The resin is thermally decomposed, and converted into a carbonized product by heating while releasing an alcohol, acetone, or the like. The carbonized product may be activated to obtain an adsorbent with high purity at high carbonization efficiency.

The inventors of the invention have found that the total volume of fine pores with diameters in a range from 0.02 to 10 $\mu$m in a carbonized product can be adjusted by finely controlling the heating conditions in the above-mentioned thermal decomposition process of a phenol resin. Successively, the inventors of the invention have found it possible to develop fine pores having diameters smaller than 20 nm and relevant to the adsorbability by activating the carbonized product while scarcely changing the fine pore structure of the diameters from 0.02 to 10 $\mu$m and accordingly to obtain an activated carbon provided with the desired fine pore structure of the diameters from 0.02 to 10 $\mu$m and the developed micro-pore structure with a diameter smaller than 0.02 $\mu$m and based on the findings, the inventors have made investigations and finally have accomplished the invention.

That is, the invention provides,

(1) an adsorbent for water treatment or medical care produced from a phenol resin as a starting material and having 0.10 to 0.60 ml/g of volume of fine pores with diameters in a range from 0.02 to 10 $\mu$m, a specific surface area in a range from 800 to 3000 m$^2$/g, and an average particle diameter in a range from 0.1 to 0.8 mm: and

(2) a method of producing an adsorbent for water treatment or medical care from a phenol resin as a starting material and having 0.10 to 0.60 ml/g of volume of fine pores with diameters in a range from 0.02 to 10 $\mu$m, a specific surface area in a range from 800 to 3000 m$^2$/g, and an average particle diameter in a range from 0.1 to 0.8 mm by conducting carbonization of the phenol resin at 350 to 550°C, then heating up to 700 to 950°C at a rate of 5 to 20°C/min, and successively carrying out activation.

The adsorbent of the invention can be produced by carbonizing a phenol resin in specified conditions and successively activating the carbonized product.

A starting material of an activated carbon to be used for the invention is a phenol resin and all kinds of phenol resins synthesized by conventionally known methods described in"14102 Chemical products", p 1031-1033 (2002) published by KAGAKUKOUGYOU NIPPOUSHA [The Chemical Daily Co., Ltd.] may be used.

Phenol resins are opaque resins with a specific gravity of 1.25 to 1.30 in term of wide definition which are obtained by condensation reaction of phenols such as phenol and cresol with aldehydes such as formaldehyde and furfural. In the present invention, a phenol resin defined in a narrow definition obtained by condensation reaction of phenol and formaldehyde is used preferably. The phenol resin may include a novolak type phenol resin to be produced using an acid catalyst and a resole type phenol resin to be produced using a basic catalyst and the resole type resin is used preferably since it is excellent in formability and easy to obtain a resin with a desired particle diameter.

The step of carbonizing the phenol resin in specified conditions is carried out by thermally decomposing the phenol resin in a proper thermal treatment apparatus. A thermal treatment apparatus to be employed may be apparatus or facilities which are capable of controlling temperature and introducing the inert gas so as to prevent occurrence of combustion of the phenol resin, a decomposition gas and the obtained thermal decomposition product. Examples to be used as the apparatus may include a fixed bed packed layer type furnace, a cross flow, vertical, fluidized bed type furnace, a rotary kiln, and a fluidized bed type furnace. Further, depending on the production scale, a starting material may be put in a container such as a crucible which may be covered with a cover and heated in an electric furnace capable of adjusting temperature.

Generally, the carbonization of the phenol resin starts at a temperature ranging from 300 to 550°C, at which the phenol resin decomposition is intensified, preferably in a range from 350 to 500°C, and more preferably in a range from 350 to 450°C. The carbonization is started by rapidly heating up to the temperature and heating is continued at a rate of 5 to 20°C/min to 700 to 950°C, at which the thermal decomposition is almost completed. The bulk density of the carbonized product obtained in this manner is about 0.65 to 0.80 g/ml although depending on the particle size.

If the carbonization starting temperature is lower than the above-mentioned temperature or the carbonization speed is too slow, carbonization of the phenol resin is gradually promoted while a decomposition gas is released slowly and simultaneously the resin itself is shrunk to give a carbonized product with an extremely small volume of fine pores with diameters in a range from 0.02 to 10 $\mu$m. In the case this carbonized product is activated but the activated carbon obtained may have a small volume of fine pores with diameters in a range from 0.02 to 10 $\mu$m. On the other hand, if the carbonization starting temperature is too high or the carbonization speed is too fast, since the thermal decomposition of the phenol resin abruptly occurs, the decomposition gas is at once released to give a carbonized product with a greatly increased volume of the fine pores with diameters in a range from 0.02 to 10 $\mu$m and in the case of activation of the carbonized product, the activated carbon to be obtained may have a low bulk density and a high volume of the fine pores with diameters in a range from 0.02 to 10 μm.

If heating treatment of the phenol resin is carried out at a temperature in a range from 200°C to 300°C, at which the carbonization is not started, for 20 minutes to 10 hours, the above-mentioned abrupt shrinkage of the resin can be suppressed and thus it is more preferable to carry out the heating treatment in production of the aimed activated carbon.

An activation method of the carbonized phenol resin is not particularly limited. The method may be carried out with an activation gas such as steam, oxygen or carbon dioxide gas at a temperature in a range generally from 700°C to 1200°C and preferably from 800°C to 1100°C as described in "Activated Carbon Industries", p. 23-37 (1974), published by "The Heavy & Chemical Industries News Agency", and a chemical activation method using phosphoric acid or zinc chloride. The activation time may be properly selected in accordance with the scale, temperature and other conditions; it is generally in a range from 10 minutes to 12 hours and preferably in a range from 30 minutes to 10 hours.

The specific surface area of the activated carbon subjected to the activation treatment is in a range generally from 800 to 3000 m²/g, preferably from 900 to 2500 m²/g, and further preferably from 950 to 2400 m²/g.

The average particle diameter of the adsorbent of the invention is in a range generally from 0.1 to 0.8 mm, preferably from 0.2 to 0.8 mm, and more preferably 0.2 to 0.5 mm. The average particle diameter here means the particle diameter measured as a value at 50% cumulative under-sieve ratio in the logarithmic normal distribution formed by plotting the particle sizes measured by a method standardized in JIS K1474.

The adsorbent with the above-mentioned particle diameter may be obtained by sieving the activated carbon subjected to the activation treatment or the adsorbent may be produced using the phenol resin previously sieved to have a prescribed particle diameter.

The volume of the fine pores with diameters in a range from 0.02 to 10 μm in the adsorbent may be measured by mercury intrusion porosimetry with a mercury porosimeter for fine pore volume measurement (e.g. AUTOPORE 9220, manufactured by MICROMERITICS). An adsorbent is charged in a sample container, and degassed under a pressure of 2.67 Pa or less for 30 minutes. Then, mercury is put in the sample container and intruding mercury in to the fine pores of the adsorbent by gradually increasing pressure (the highest pressure = 414 MPa). The fine pore volume distribution of adsorbent is measured by the following respective calculation equations based on the relation of the pressure and the intrusion amount of mercury. A pore diameter can be calculated as follows. When mercury is forced into a cylindrical micropore having a diameter (D) by applying a pressure (P), a surface tension of mercury is balanced with pressure acting on a section of the micropore, and thus, a following equation is held:

$$-\pi D \gamma \, \cos\theta = \pi(D/2)^2 \times P$$

wherein γ is a surface tension of mercury; and θ is a contact angle of mercury and a wall of the micropore. Therefore, a following equation:

$$D = (-\,4\,\gamma\,\cos\theta)/P$$

is held. In this specification, the surface tension γ of mercury is set to be 480 dyne/cm; the contact angle θ of mercury and carbon is set to be 140°; the unit of the pressure P is set to be Pa; and the unit of the fine pore diameter D is set to be μm to calculate the correlation between the pressure P and the fine pore diameter D as the following equation:

$$D = 1.47 \times 10^2/P.$$

The volume of the fine pores with pore diameters in a range from 0.02 to 10 μm in the invention is equivalent to the volume of mercury intruded at mercury intrusion pressure of $1.47 \times 10^2$ Pa to $7.35 \times 10^2$ Pa.

The fine pores with the pore diameter in a range from 0.02 to 10 μm do not directly contribute to adsorption of a substance, but form transfer paths of the substance to be adsorbed in fine pores. That is, if the number of the fine pores in the above-mentioned range is too small, the movement of the adsorbed substance is disturbed to result in decrease of the adsorption efficiency. On the contrary, if the number of the fine pores in the above-mentioned range is too large, the adsorbent is rather significantly porous and the adsorption amount of the adsorbent per unit volume is decreased to lower the adsorbability in the case the adsorbent is packed in a certain volume in, for example, an adsorption column, an adsorption tower, or an adsorption pond. As described, with respect to the adsorption speed of the adsorbent, it has been found that the ratio of the volume of the fine pore with diameters in a range from 0.02 to 10 μm in the

adsorbent is a very important factor.

The volume of the fine pore with diameters in a range from 0.02 to 10 μm in the adsorbent is in a range from 0.10 to 0.60 ml/g, preferably from 0.10 to 0.45 ml/g, more preferably from 0.10 to 0.40 ml/g, and even more from 0.10 to 0.30 ml/g. The bulk density of the adsorbent is in a range generally from 0.33 to 0.80 g/ml and more preferably from 0.40 to 0.70 g/ml. The adsorbent having the fine pores with diameters in a range from 0.02 to 10 μm has an outstandingly high adsorption rate.

Effects of the Invention

The adsorbent of the invention can quickly and efficiently adsorb and remove an impurity and a low molecular compound such as a coloring material in water and is therefore useful as an adsorbent for water purifiers and an adsorbent for adsorbing a specified substance in digestive tracts by oral administration.

Best Mode for Carrying Out the Invention

Hereinafter, the invention will be described further in detail with reference to Reference Examples, Examples, Comparative Examples, and Test Examples; however it is not intended that the invention be limited to the described embodiments.

Example 1

A commercially available spherical phenol-formaldehyde resin was sieved to adjust the particle size in a range from 0.71 to 0.212 mm. Successively, 400 g of the resin was put in an externally heating type 2 L capacity rotary kiln adjusted previously at 400°C and while nitrogen gas was passed at 5 L/min. The resin was heated to 850°C at 10°C/min heating rate and then the heated resin was discharged to nitrogen gas atmosphere and cooled to a room temperature. Next, 180 g of the obtained carbonized product was put in an externally heating type 2 L capacity rotary kiln adjusted at 900°C and activated by passing steam at 180 g/hr and after 6 hours, the product was discharged to nitrogen gas atmosphere and cooled to a room temperature to obtain an adsorbent No. 1. The obtained adsorbent had 1780 $m^2$/g of a specific surface area, 0.448 g/ml of a bulk density, and 0.18 ml/g of a volume of fine pores with diameters in a range from 0.02 to 10 μm.

Example 2

An adsorbent No. 2 was obtained in the same manner as Example 1, except that the carbonization starting temperature was changed to 350°C. The specific surface area and the volume of fine pores with diameters in a range from 0.02 to 10 μm of the obtained adsorbent were 1790 $m^2$/g and 0.11 ml/g, respectively.

Example 3

An adsorbent No. 3 was obtained in the same manner as Example 1, except that the carbonization started at a temperature of 450°C. The specific surface area and the volume of fine pores with diameters in a range from 0.02 to 10 μm of the obtained adsorbent were 1770 $m^2$/g and 0.26 ml/g, respectively.

Example 4

An adsorbent No. 4 was obtained in the same manner as Example 1, except that the activation time of the carbonized product was changed to 3 hours. The specific surface area and the volume of fine pores with diameters in a range from 0.02 to 10 μm of the obtained adsorbent were 1250 $m^2$/g and 0.18 ml/g, respectively.

Example 5

An adsorbent No. 5 was obtained in the same manner as Example 1, except that the activation time of the carbonized product was changed to 9 hours. The specific surface area and the volume of fine pores with diameters in a range from 0.02 to 10 μm of the obtained adsorbent were 2400 $m^2$/g and 0.22 ml/g, respectively.

Example 6

An adsorbent No. 6 was obtained in the same manner as Example 1, except that the activation time of the carbonized product was changed to 1 hour. The specific surface area and the volume of fine pores with diameters in a range from 0.02 to 10 $\mu$m of the obtained adsorbent were 950 m$^2$/g and 0.13 ml/g, respectively.

Example 7

An adsorbent No. 7 was obtained in the same manner as Example 1, except that the activation time of the carbonized product was changed to 2 hours. The specific surface area and the volume of fine pores with diameters in a range from 0.02 to 10 $\mu$m of the obtained adsorbent were 1090 m$^2$/g and 0.14 ml/g, respectively.

Example 8

An adsorbent No. 8 was obtained in the same manner as Example 1, except that the heating rate for carbonization was changed to 7.5°C/min. The specific surface area and the volume of fine pores with diameters in a range from 0.02 to 10 $\mu$m of the obtained adsorbent were 1800 m$^2$/g and 0.13 ml/g, respectively.

Example 9

An adsorbent No. 9 was obtained in the same manner as Example 1, except that the carbonization rate was changed to 30°C/min. The specific surface area and the volume of fine pores with diameters in a range from 0.02 to 10 $\mu$m of the obtained adsorbent were 1760 m$^2$/g and 0.35 ml/g, respectively.

Example 10

An adsorbent No. 10 was obtained in the same manner as Example 1, except that the carbonization starting temperature was changed to 600°C. The specific surface area and the volume of fine pores with diameters in a range from 0.02 to 10 $\mu$m of the obtained adsorbent were 1770 m$^2$/g and 0.40 ml/g, respectively.

Example 11

An adsorbent No. 11 was obtained in the same manner as Example 2, except that 400 g of the phenol resin was put in an externally heating type 2 L capacity rotary kiln adjusted previously at 200°C and while nitrogen gas was passed at 5 L/min, the resin was kept for 8 hours for heat treatment and then the heated resin was taken out once and cooled to a room temperature. The specific surface area and the volume of fine pores with diameters in a range from 0.02 to 10 $\mu$m of the obtained adsorbent were 1780 m$^2$/g and 0.16 ml/g, respectively.

Example 12

An adsorbent No. 12 was obtained in the same manner as Example 2, except that 400 g of the phenol resin was put in an externally heating type 2 L capacity rotary kiln adjusted previously at 300°C and while nitrogen gas was passed at 5 L/min, the resin was kept for 3.5 hours for heat treatment and then the heated resin was taken out once and cooled to a room temperature. The specific surface area and the volume of fine pores with diameters in a range from 0.02 to 10 $\mu$m of the obtained adsorbent were 1800 m$^2$/g and 0.15 ml/g, respectively.

Example 13

An adsorbent No. 13 was obtained in the same manner as Example 1, except that 400 g of the phenol resin was put in an externally heating type 2 L capacity rotary kiln adjusted previously at 200°C and while nitrogen gas was passed at 5 L/min, the resin was heated to 300°C in 20 minutes and kept at the temperature for 40 minutes and heated to 850°C in 50 minutes for carbonization. The specific surface area and the volume of fine pores with diameters in a range from 0.02 to 10 $\mu$m of the obtained adsorbent were 1810 m$^2$/g and 0.17 ml/g, respectively.

Comparative Example 1

An adsorbent No. 14 was obtained in the same manner as Example 1, except that the carbonization starting temperature was changed to 200°C and the heating time for carbonization was changed to 65 minutes. The specific surface area and the volume of fine pores with diameters in a range from 0.02 to 10 $\mu$m of the obtained adsorbent were 1750 m$^2$/g and 0.02 ml/g, respectively.

Comparative Example 2

An adsorbent No. 15 was obtained in the same manner as Example 1, except that the carbonization rate was changed to 2.5°C/min. The specific surface area and the volume of fine pores with diameters in a range from 0.02 to 10 $\mu$m of the obtained adsorbent were 1780 $m^2$/g and 0.08 ml/g, respectively.

Comparative Example 3

An adsorbent No. 16 was obtained from a carbonized product of coconut shell produced from Zamboanga Province, Philippines in place of the phenol resin and activating the carbonized product with steam for 3 hours. The specific surface area and the volume of fine pores with diameters in a range from 0.02 to 10 $\mu$m of the obtained adsorbent were 1680 $m^2$/g and 0.36 ml/g, respectively.

Comparative Example 4

An adsorbent No. 17 was obtained from a phenol resin produced in the method described in Example 2 of JP-A No. 2004-24414 as a starting material. The specific surface area and the volume of fine pores with diameters in a range from 0.02 to 10 $\mu$m of the obtained adsorbent were 1286 $m^2$/g and 0.02 ml/g, respectively.

Comparative Example 5

An adsorbent No. 18 was prepared by the method described in Example 1 of WO No. 2004-039381. The specific surface area and the volume of fine pores with diameters in a range from 0.02 to 10 $\mu$m of the obtained adsorbent were 1740 $m^2$/g and 0.07 ml/g, respectively.
The production conditions of the above-mentioned adsorbents and the properties of the obtained adsorbents are collectively shown in Table 1.
Table 1
Phenol resin properties and production methods of adsorbents

| Example ,Comparative Example No. | | Example 1 | Example 2 | Example 3 | Example 4 | Example 5 | Example 6 | Example 7 | Example 8 | Example 9 |
|---|---|---|---|---|---|---|---|---|---|---|
| Adsorbent No. | | No. 1 | No. 2 | No. 3 | No. 4 | No. 5 | No. 6 | No. 7 | No. 8 | No. 9 |
| Heat treatment starting temperature | °C | - | - | - | - | - | - | - | - | - |
| Heating time | min. | - | - | - | - | - | - | - | - | - |
| Heat treatment finishing temperature | °C | - | - | - | - | - | - | - | - | - |
| Retention time | h | - | - | - | - | - | - | - | - | - |
| Carbonization starting temperature | °C | 400 | 350 | 450 | 400 | 400 | 400 | 400 | 400 | 400 |
| Carbonization time | min. | 45 | 50 | 40 | 45 | 45 | 45 | 45 | 60 | 15 |
| Carbonization rate | °C/min. | 10 | 10 | 10 | 10 | 10 | 10 | 10 | 7.5 | 30 |
| Carbonization finishing temperature | °C | 850 | 850 | 850 | 850 | 850 | 850 | 850 | 850 | 850 |
| Specific surface area | $m^2$/g | 1780 | 1790 | 1770 | 1250 | 2400 | 950 | 1090 | 1800 | 1760 |
| Volume of fine pores with diameters of 20 to 10000 nm | ml/g | 0.18 | 0.11 | 0.26 | 0.18 | 0.22 | 0.13 | 0.14 | 0.13 | 0.35 |
| Bulk density | g/ml | 0.448 | 0.472 | 0.421 | 0.554 | 0.345 | 0.732 | 0.662 | 0.465 | 0.402 |

Continued

| Example ,Comparative Example No. | | Example 10 | Example 11 | Example 12 | Example 13 | Comparative Example 1 | Comparative Example 2 | Comparative Example 3 | Comparative Example 4 | Comparative Example 5 |
|---|---|---|---|---|---|---|---|---|---|---|
| Adsorbent No. | | No. 10 | No. 11 | No. 12 | No. 13 | No. 14 | No. 15 | No. 16 | No. 17 | No. 18 |
| Heat treatment starting temperature | °C | · | 200 | 300 | 200 | · | · | · | · | · |
| Heating time | min. | · | · | · | 20 | · | · | · | · | · |
| Heat treatment finishing temperature | °C | · | 200 | 300 | 30 | · | · | · | · | · |
| Retention time | h | · | 480 | 210 | 40 | · | · | · | · | · |
| Carbonization starting temperature | °C | 600 | 350 | 350 | 300 | 200 | 400 | · | · | · |
| Carbonization time | min. | 25 | 50 | 50 | 55 | 65 | 180 | · | · | · |
| Carbonization rate | °C/min. | 10 | 10 | 10 | 10 | 10 | 2.5 | · | · | · |
| Carbonization finishing temperature | °C | 850 | 850 | 850 | 850 | 850 | 850 | · | · | · |
| Specific surface area | m²/g | 1770 | 1780 | 1800 | 1810 | 1750 | 1780 | 1680 | 1286 | 1740 |
| Volume of fine pores with diameters of 20 to 10000 nm | ml/g | 0.40 | 0.16 | 0.15 | 0.17 | 0.02 | 0.08 | 0.36 | 0.02 | 0.07 |
| Bulk density | g/ml | 0.374 | 0.470 | 0.468 | 0.455 | 0.550 | 0.502 | 0.404 | 0.630 | 0.534 |

Test Example 1

Comparison of DL-β-aminoisobutyric acid adsorption rate

Each adsorbent previously dried at 105°C in an amount of 5.0 ml was measured by a measuring cylinder and put in a 100 ml conical flask. Fifty ml of an aqueous DL-β-aminoisobutyric acid solution which was prepared by dissolving 100.0 mg of DL-β-aminoisobutyric acid in a 0.05 mol/L phosphoric acid buffer solution (pH value 7.4) and precisely adjusting the volume to 1 L was added and the resulting conical flask. The mixture was set in a thermostat shaker kept at 37±2°C and shaken at 160 rpm with 40 mm amplitude. The supernatant in volume of 1 ml was sampled by a measuring pipette after 10 minutes, 20 minutes, 30 minutes, and 60 minutes from starting of the shaking and DL-β-aminoisobutyric acid was quantitatively measured by liquid chromatography.
The DL-β-aminoisobutyric acid adsorption ratio was calculated according to the following equation. The experimental results are shown in Table 2.

$$\text{DL-}\beta\text{-aminoisobutyric acid adsorption ratio (\%)} = 100 - \text{(concentration before shaking - concentration after shaking)} \div \text{(concentration before shaking)} \times 100.$$

Table 2

Aminoisobutyric acid adsorption ratio

| Example , Comparative Example No. | | Example 1 | Example 2 | Example 3 | Example 4 | Example 5 | Example 6 | Example 7 | Example 8 | Example 9 |
|---|---|---|---|---|---|---|---|---|---|---|
| Adsorbent number | | No. 1 | No. 2 | No. 3 | No. 4 | No. 5 | No. 6 | No. 7 | No. 8 | No. 9 |
| Weight of 5 ml sample | g | 2.24 | 2.36 | 2.11 | 2.77 | 1.73 | 3.66 | 3.31 | 2.33 | 2.01 |
| Aminoisobutyric acid adsorption ratio | | | | | | | | | | |
| After 10 minutes | % | 48 | 45 | 52 | 49 | 54 | 44 | 45 | 46 | 51 |
| After 20 minutes | % | 60 | 57 | 65 | 57 | 63 | 53 | 54 | 55 | 64 |
| After 30 minutes | % | 70 | 65 | 71 | 72 | 72 | 61 | 69 | 64 | 75 |
| After 60 minutes | % | 84 | 85 | 83 | 85 | 82 | 80 | 81 | 84 | 80 |

Continued

| Example ,Comparative Example No. | | Example 10 | Example 11 | Example 12 | Example 13 | Comparative Example 1 | Comparative Example 2 | Comparative Example 3 | Comparative Example 4 | Comparative Example 5 |
|---|---|---|---|---|---|---|---|---|---|---|
| Adsorbent number | | No. 10 | No. 11 | No. 12 | No. 13 | No. 14 | No. 15 | No. 16 | No. 17 | No. 18 |
| Weight of 5 ml sample | g | 1.87 | 2.35 | 2.34 | 2.28 | 2.75 | 2.51 | 2.02 | 3.15 | 2.67 |
| Aminoisobutyric acid adsorption ratio | | | | | | | | | | |
| After 10 minutes | % | 54 | 50 | 49 | 48 | 25 | 32 | 40 | 26 | 32 |
| After 20 minutes | % | 63 | 64 | 63 | 62 | 38 | 45 | 47 | 30 | 41 |
| After 30 minutes | % | 70 | 72 | 72 | 71 | 51 | 50 | 55 | 36 | 49 |
| After 60 minutes | % | 81 | 86 | 85 | 83 | 69 | 68 | 59 | 44 | 57 |

As is clear from Table 2, the adsorbents within the scope of the invention showed high adsorption ratio around 50% only after 10 minutes from starting of the adsorption and many of the adsorbents showed adsorption ratio exceeding 60% after 20 minutes and all showed 80% or higher adsorption ratio after 60 minutes. On the other hand, the activated carbons with small volumes of fine pores with diameters in a range from 0.02 to 10 $\mu$m as those of Comparative Examples 1, 2, 4, and 5 (adsorbents Nos. 14, 15, 17 and 18) showed low adsorbability at any time point from starting of adsorption as compared with that of adsorbents within a scope of the invention and there was no adsorbent having adsorption ratio exceeding 70% even after 60 minutes. Further, the adsorbent produced from activated carbon of coconut shell (Comparative Example 3, adsorbent No. 16) was found having low adsorbability due to the difference of the starting material. As described, since the adsorbents of Examples of the present invention are capable of adsorbing and removing object substances to be adsorbed within a short time in digestive tracts in the case they are used as pharmaceutical compositions, they can lessen the absorption of these substances in living bodies (vascular tracts and internal organs) and are thus very useful for pharmaceutical compositions.

Test Example 2

Decolorization Test of Aqueous Methylene Blue Solution by Passing Column

An aqueous thin Methylene Blue solution [obtained by dissolving 0.24 g (based on the weight in dry state) of Methylene Blue in a phosphoric acid buffer solution at pH 7.4 and adjusting the volume to be 1 L] was passed at 5 ml/min through a glass column with an inner diameter of 2.5 cm and filled with 60 ml of each adsorbent. The water taken out of the outlet of the column was sampled after every 1 hour from the starting of passing to measure the Methylene Blue concentration. The passing volume until the time when the Methylene Blue concentration exceeds 1.2 mg/l was determined to be the Methylene Blue decolorization capability. The results were shown in Table 3.
Table 3

Methylene Blue decolorization capability

| Example ,Comparative Example No. | | Example 1 | Example 2 | Example 3 | Example 4 | Example 5 | Example 6 | Example 7 | Example 8 | Example 9 |
|---|---|---|---|---|---|---|---|---|---|---|
| Adsorbent No. | | No. 1 | No. 2 | No. 3 | No. 4 | No. 5 | No. 6 | No. 7 | No. 8 | No. 9 |
| Column-filling amount | g | 23.7 | 24.9 | 22.2 | 29.3 | 18.2 | 38.6 | 35.0 | 24.6 | 21.2 |
| Methylene Blue decolorization capability | L | 25 | 23 | 20 | 21 | 23 | 20 | 22 | 24 | 21 |

Continued

| Example ,Comparative Example No. | | Example 10 | Example 11 | Example 12 | Example 13 | Comparative Example 1 | Comparative Example 2 | Comparative Example 3 | Comparative Example 4 | Comparative Example 5 |
|---|---|---|---|---|---|---|---|---|---|---|
| Adsorbent No. | | No. 10 | No. 11 | No. 12 | No. 13 | No. 14 | No. 15 | No. 16 | No. 17 | No. 18 |
| Column-filling amount | g | 19.7 | 24.8 | 24.7 | 24.0 | 29.0 | 26.5 | 21.3 | 33.3 | 28.2 |
| Methylene Blue decolorization capability | L | 20 | 24 | 23 | 25 | 10 | 12 | 16 | 10 | 16 |

EP 1 757 362 A1

As is clear from Table 3, the adsorbents of Examples of the invention showed higher dynamic adsorbability as compared with the adsorbents of Comparative Examples. That is, having small volumes of fine pores with diameters in a range from 0.02 to 10 $\mu$m and slow adsorption rates, the adsorbents of Comparative Examples 1, 2, 3, and 5 (Adsorbent Nos. 14, 15, 17 and 18) were deteriorated in the decolorization effect of Methylene Blue within a short time in the case of the passing test and were found having inferior decolorization capability. On the other hand, since the adsorbents of the invention were adjusted to have the volumes of fine pores with diameters in a range from 0.02 to 10 $\mu$m, in the case they were packed in an adsorption apparatus such as a packed column, they showed high adsorbability per unit volume.

Industrial Applicability

Since an adsorbent of the invention not only efficiently removes an impurity such as a coloring material, an organic or inorganic toxic substance contaminating water, or a low molecular weight metabolism product existing in living bodies per unit volume but also has high adsorption rate and high adsorbability, it can be used preferably as an adsorbent for water treatment and an adsorbent for medical care. As the adsorbent for water treatment, it is useful as an adsorbent for a compact water purifier. As the adsorbent for medical care, about 0.1 to 5 g of the adsorbent may be packed in a bag or filled in a capsule and administered orally one to three times a day to a patient. The adsorbent is particularly efficacious for a pharmaceutical for renal insufficiency remedy.

**Claims**

1. An adsorbent for water treatment or medical care produced from a phenol resin as a starting material and having 0.10 to 0.60 ml/g of volume of fine pores with diameters in a range from 0.02 to 10 $\mu$m, a specific surface area in a range from 800 to 3000 m$^2$/g, and an average particle diameter in a range from 0.1 to 0.8 mm.

2. A method of producing an adsorbent for water treatment or medical care from a phenol resin as a starting material and having 0.10 to 0.60 ml/g of volume of fine pores with diameters in a range from 0.02 to 10 $\mu$m, a specific surface area in a range from 800 to 3000 m$^2$/g, and an average particle diameter in a range from 0.1 to 0.8 mm by conducting carbonization of the phenol resin at 350 to 550°C, then heating up to 700 to 950°C at a rate of 5 to 20°C/min, and successively carrying out activation.

EP 1 757 362 A1

<table>
<tr><td colspan="2" align="center"><b>INTERNATIONAL SEARCH REPORT</b></td><td>International application No.<br>PCT/JP2005/009748</td></tr>
</table>

A. CLASSIFICATION OF SUBJECT MATTER
  Int.Cl⁷  B01J20/20, A61K31/775, B01J20/30

According to International Patent Classification (IPC) or to both national classification and IPC

B. FIELDS SEARCHED

Minimum documentation searched (classification system followed by classification symbols)
  Int.Cl⁷  B01J20/20, A61K31/775, B01J20/30, C02F1/28

Documentation searched other than minimum documentation to the extent that such documents are included in the fields searched
  Jitsuyo Shinan Koho        1922-1996    Jitsuyo Shinan Toroku Koho    1996-2005
  Kokai Jitsuyo Shinan Koho  1971-2005    Toroku Jitsuyo Shinan Koho    1994-2005

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 9-110409 A  (Kanebo, Ltd.),<br>28 April, 1997 (28.04.97),<br>Claims; Par. Nos. [0001], [0020]; examples<br>(Family: none) | 1 |
| X | JP 54-89010 A  (Kureha Chemical Industry Co., Ltd.),<br>14 July, 1979 (14.07.79),<br>Claims; page 2, upper right column<br>& GB 2012257 A        & DE 2855825 A<br>& FR 2413092 A | 1 |
| X | JP 8-208491 A  (Kureha Chemical Industry Co., Ltd.),<br>13 August, 1996 (13.08.96),<br>Claims; Par. Nos. [0005], [0007]<br>& EP 711561 A2        & US 5573761 A | 1 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
  20 June, 2005 (20.06.05)

Date of mailing of the international search report
  05 July, 2005 (05.07.05)

Name and mailing address of the ISA/
  Japanese Patent Office

Authorized officer

Facsimile No.

Telephone No.

Form PCT/ISA/210 (second sheet) (January 2004)

13

EP 1 757 362 A1

| INTERNATIONAL SEARCH REPORT | International application No. |
|---|---|
| | PCT/JP2005/009748 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2004/039381 A1  (Kureha Chemical Industry Co., Ltd.), 13 May, 2004 (13.05.04), Claims; pages 7 to 8 (Family: none) | 1,2 |

Form PCT/ISA/210 (continuation of second sheet) (January 2004)

14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S6211611 A
- JP 2002308785 A
- WO 2004039381 A1
- JP 2004244414 A
- JP 2004024414 A
- WO 2004039381 A

**Non-patent literature cited in the description**

- 14102 Chemical products. KAGAKUKOUGYOU NIPPOUSHA [The Chemical Daily Co., Ltd, 2002, 1031-1033
- Activated Carbon Industries. The Heavy & Chemical Industries News Agency, 1974, 23-37